# EUROPÄISCHE PATENTSCHRIFT

(11) **EP 0 761 167 B1**
(45) Veröffentlichungstag und Bekanntmachung des Hinweises auf die Patenterteilung: **20.11.2002**
(21) Anmeldenummer: 96114003.5
(22) Anmeldetag: 30.08.1996
(51) Int. Cl.: A61B 6/14

(54) **Röntgendiagnostikeinrichtung mit einer Positioniervorrichtung für einen Strahlensender und einen Strahlenempfänger**
X-ray diagnostic device comprising positioning means for a radiation generator and radiation receiver
Appareil diagnostique à rayons comportant un dispositif de positionnement pour générateur à rayonnement et récepteur à rayonnement

(30) Priorität: 12.09.1995 DE 19533716
(43) Veröffentlichungstag der Anmeldung: 12.03.1997
(73) Patentinhaber: Sirona Dental Systems GmbH, 64625 Bensheim (DE)
(72) Erfinder: Plötz, Josef, Dr., 64625 Bensheim (DE)
(74) Vertreter: Sommer, Peter

(56) Entgegenhaltungen:
- DE-A- 4 414 689
- DE-A- 4 440 376

## Beschreibung

In der dentalen Röntgendiagnostik sind Positioniervorrichtungen für einen Röntgenfilm bekannt. Der Röntgenfilm wird hierbei im Mund angeordnet und über die Positioniervorrichtung in Bezug zu einem Röntgenstrahler ausgerichtet. Eine solche Vorrichtung ist beispielsweise aus der Literaturstelle Journal of the American Dental Association (J.A.D.A.), 1967, Folge 75, Seite 1361 bis 1368, Updegrave W.J., mit dem Titel "Simplified and standarized bisecting-angle technic for dental radiography" bekannt. Durch diese Vorrichtung soll sichergestellt werden, daß ein von einem Röntgenstrahler ausgehendes Strahlenbündel möglichst senkrecht auf die Filmebene auftrifft. Eine Kopplung zwischen dem Filmhalter und dem Röntgenstrahler ist weder erwähnt noch vorgesehen.

Aus der DE-A- 44 14 689 (veröffentlicht nach dem Prioritätsdatum der vorliegenden Anmeldung) ist eine Röntgendiagnostikeinrichtung mit einer Positioniervorrichtung für einen Strahlensender bekannt. Durch Ankopplung des Strahlensenders an diese Positioniervorrichtung wird sichergestellt, daß ein Bezugsstrahl eines emittierten Strahlenbündels eine in Bezug zur Positioniervorrichtung ortsfeste Bezugsachse in einem spitzen Winkel α schneidet. Es ist ferner bekannt, daß der Strahlensender durch Ankopplung Positionen entlang eines Kreisbogens einnehmen kann, wobei das Zentrum des Kreisbogens auf der Bezugsachse liegt. Es ist ferner bekannt, an die Vorrichtung einen Tragarm für einen Strahlungswandler anzukoppeln, der den Abstand eines zu untersuchenden Objektes zur Vorrichtung und damit zum Fokus des Strahlensenders vorgibt. Der Abstand ist hierbei so gewählt, daß der Bezugsstrahl des Strahlenbündels auf das Zentrum des Strahlungswandlers trifft.

Aus der DE 44 40 376 ist eine Röntgenvorrichtung bekannt, bei welcher die Röntgenstrahlenquelle um ein Zentrum einer planigrafischen Ebene verstellt wird. Synchron zu dieser Bewegung wird eine Röntgenfilmkassette in einer Richtung entgegengesetzt der Bewegungsrichtung der Röntgenstrahlenquelle parallel verstellt. Aufgrund dieser Steuerung wird das Röntgenbild des gleichen Bereichs der planigrafischen Ebene 1 immer an der gleichen Position der Filmkassette gebildet.

Aufgabe der Erfindung ist es, eine Röntgendiagnostikeinrichtung der eingangs genannten Art so auszuführen, daß diese insbesondere für die Erstellung von Tomosyntheseaufnahmen, insbesondere zur Diagnose relativ kleiner Bereiche in größeren Objekten, beispielsweise eines Extremitätengelenks, von Teilen des Schädels, insbesondere der Zähne und Kiefergelenke, geeignet ist. Diese Vorrichtung soll zudem unkompliziert in der Handhabung und kostengünstig herstellbar sein. Sie soll insbesondere so ausgeführt sein, daß bei einer Dentaluntersuchung der Strahlenempfänger nicht im Mund eines Patienten angeordnet werden muß.

Die Aufgabe wird erfindungsgemäß durch eine Röntgendiagnostikeinrichtung nach dem Gegenstand des Patentanspruches 1 gelöst.

Vorteil der Erfindung ist, daß sowohl für den Strahlensender als auch für den Strahlenempfänger jeweils eine Positioniervorrichtung vorgesehen ist, die eine gemeinsame Bezugsachse haben. Der Abstand zwischen dem Strahlensender und dem Strahlenempfänger ist somit vorgegeben. Es ist zur Erstellung von Tomosyntheseaufnahmen von Vorteil, wenn bei einer Ankopplung des Strahlensenders an die erste Positioniervorrichtung ein Bezugsstrahl eines vom Strahlensender emittierten Strahlenbündels die gemeinsame Bezugsachse in einem spitzen Winkel schneidet und zumindest annähernd zentral auf den Strahlenempfänger trifft. Der Strahlenempfänger kann somit Signale erzeugen, die in zum Bezugsstrahl lateralen Richtungen ortsaufgelöst sind, so daß aufgrund dieser Signale Schichtbilder in vorbestimmten Ebenen berechnet werden können.

Weitere Vorteile und Einzelheiten der Erfindung ergeben sich aus der nachfolgenden Beschreibung eines Ausführungsbeispieles anhand der Zeichnungen in Verbindung mit den Unteransprüchen.

Es zeigt:
- FIG 1: in prinzipieller Weise eine Röntgendiagnostikeinrichtung nach der Erfindung und
- FIG 2: eine Steuereinrichtung der Röntgendiagnostikeinrichtung nach FIG 1

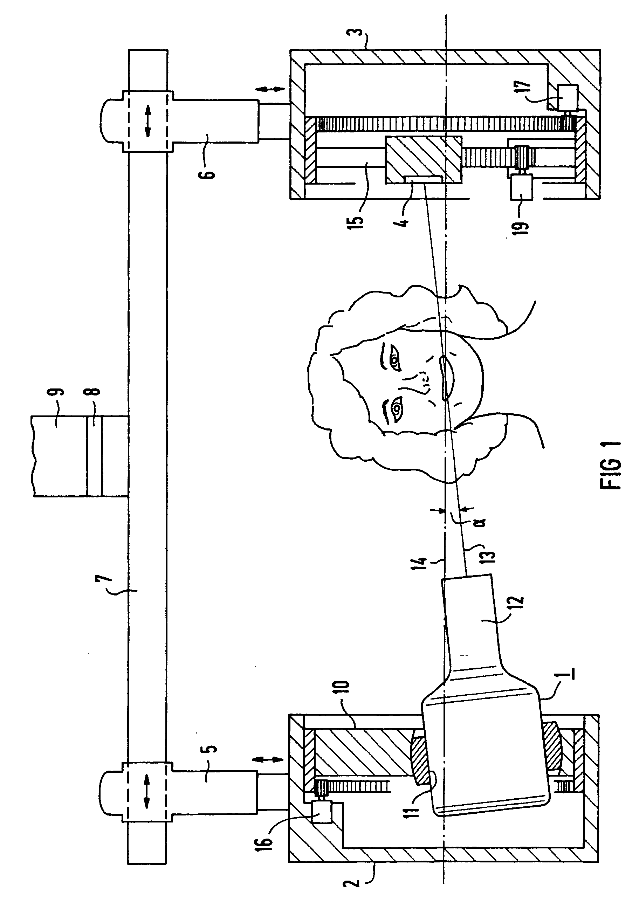

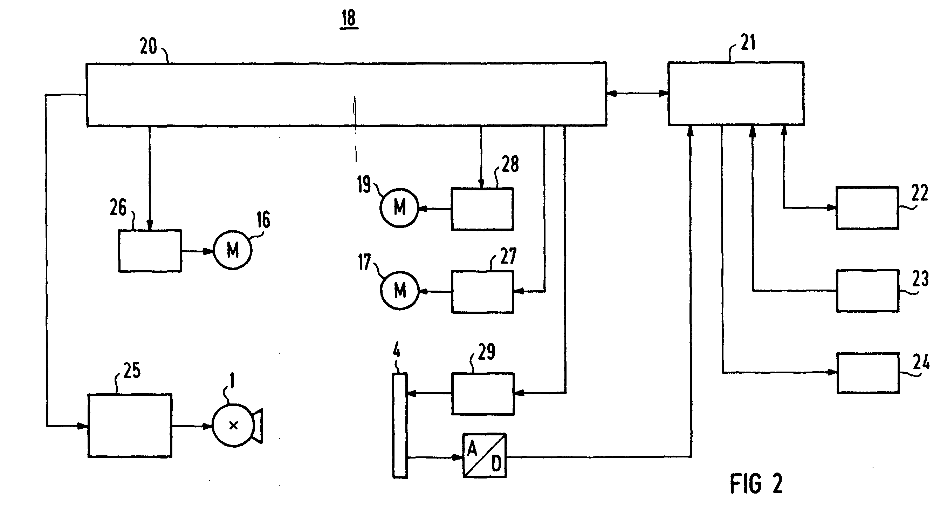

Bei der in der FIG 1 nur in prinzipieller Weise dargestellten Röntgendiagnostikeinrichtung ist eine erste Positioniervorrichtung für einen Strahlensender 1 mit dem Bezugszeichen 2 gekennzeichnet. Eine zweite Positioniervorrichtung 3 für einen Strahlenempfänger 4 ist erfindungsgemäß vorgesehen. Die Positioniervorrichtungen 2,3 sind jeweils an einer Anlenkung 5,6 gehalten, über die sie höhenverstellbar und entlang eines Trägers 7 verstellbar sind. Der Träger 7 lagert über ein Drehgelenk 8 und eine Säule 9 an der Decke eines Raumes. Die erste Positioniervorrichtung 2 kann ein scheibenförmiges Positionierelement 10, beispielsweise eine Taumelscheibe, mit einer Ausnehmung 11 zur Aufnahme des Gehäuses oder des Tubusses 12 des Strahlensenders 1 aufweisen. Bei Ankopplung des Strahlensenders 1 an das scheibenförmige Positionierelement 10 über die Ausnehmung 11 wird erreicht, daß ein Bezugsstrahl 13, beispielsweise der Zentralstrahl, eines vom Strahlensender 1 ausgesandten Strahlenbündels eine den Positioniervorrichtungen 2,3 gemeinsame Bezugsachse 14 in einem spitzen Winkel α schneidet. Die Bezugsachse 14 liegt hierbei beispielsweise im zentralen Bereich des scheibenförmigen Positionierelementes 10 der ersten Positioniervorrichtung 2 und eines zweiten scheibenförmigen Positionierelementes 15, an dem der Strahlenempfänger 4 gelagert ist. Die scheibenförmigen Positionierelemente 10,15 sind derart an ihren Anlenkungen 5,6 gelagert, daß sie um die gemeinsame Bezugsachse 14 und über jeweils einen vorgesehenen Antrieb 16,17 verstellbar sind. Über eine Steuereinrichtung 18, die nachfolgend in der FIG 2 erläutert wird, wird der Strahlenempfänger 4 über den Antrieb 17 in eine solche Position verstellt, daß der Bezugsstrahl 13 zumindest annähernd zentral auf seine strahlendetektierende Fläche auftrifft.

Beim Ausführungsbeispiel ist der Strahlenempfänger 4 an ein scheibenförmiges Positionierelement 15 ankoppelbar, das über einen Antrieb 17 um die Bezugsachse 14 und über einen weiteren Antrieb 19 im Abstand zur Bezugsachse 14 verstellbar ist. Durch Ansteuerung der Antriebe 16,17,19 kann der Strahlensender 1 und der Strahlenempfänger 4 Positionen entlang eines Kreisbogens einnehmen und der Radius des vom Strahlenempfänger 4 beschriebenen Kreisbogens variiert werden.

Gleichwirkend ist jedoch auch eine Anordnung, die ein scheiben- oder ringförmiges Element mit mehreren Ausnehmungen entlang eines Kreisbogens aufweist, in die der Tubus 12 einkoppelbar ist. Hierbei ist es jedoch notwendig, daß dann Detektorelemente vorgesehen sind, die die jeweilige Position des Strahlensenders 1 detektieren und ein dementsprechendes Signal an eine Steuereinheit zur Verstellung des Strahlenempfängers 4 liefert. Im Rahmen der Erfindung ist es natürlich auch möglich, eine Mechanik so auszubilden, daß der Strahlensender 1 und der Strahlenempfänger 4 jeweils so positioniert werden, daß bei einer Ortsveränderung beispielsweise des Strahlensenders 1 eine entsprechende Ortsveränderung des Strahlenempfängers 4 in der Art erfolgt, daß der Bezugsstrahl 13 die gemeinsame Bezugsachse 14 in einem spitzen Winkel α schneidet.

Bei einer Variante der Erfindung sind die erste und zweite Positioniervorrichtung 2,3 so ausgeführt, daß der Abstand des Strahlensenders 1 bzw. des Strahlenempfängers 4 zur gemeinsamen Bezugsachse 14 verändert werden kann.

Vorzugsweise sind die Abmessungen des Strahlenempfängers (4) kleiner als der mittlere Abstand zwischen der Bezugsachse (14) und dem Bezugsstrahl (13) am Ort des Strahlenempfängers (4) in den verschiedenen, vom Strahlensender (1) eingenommenen Positionen und wobei der Strahlenempfänger (4) so positioniert wird, daß bei den verschiedenen, vom Strahlensender (1) eingenommenen Positionen der Bezugsstrahl (13) jeweils zumindest annähernd auf das Zentrum des Strahlenempfängers (4) trifft.

In der FIG 2 ist eine Steuereinrichtung 18 für die Antriebe 16,17,19 gezeigt, die einen Mikrokontroller 20 und einen zugeordneten Rechner 21 aufweist, dem ein Speicher 22, Bedienelemente 23 und eine Ausgabeeinheit 24 zugeordnet ist. Über den Mikrokontroller 20 wird eine Spannungsversorgungseinrichtung 25 für den Strahlensender 1, die Antriebe 16,17,19 über eine jeweilige Ansteuereinrichtung 26,27,28 und eine Elektronik 29 für den Strahlenempfänger 4 angesteuert. Es ist somit möglich, die Position des Strahlensenders 1 und die des Strahlenempfängers 4 elektronisch und/oder durch Software gesteuert, einzustellen.

## Patentansprüche

1. Röntgendiagnostikeinrichtung mit einer ersten Positioniervorrichtung (2) mit einem Strahlensender (1) und mit einer zweiten Positioniervorrichtung (3) mit einem Strahlenempfänger (4), die einen Abstand zueinander haben, wobei die Positioniervorrichtungen (2,3) eine gemeinsame Bezugsachse (14) haben, wobei bei einer Ankopplung des Strahlensenders (1) an die erste Positioniervorrichtung (2) der Bezugsstrahl (13) eines vom Strahlensender (1) emittierten Strahlenbündels die gemeinsame Bezugsachse (14) in einem spitzen Winkel α schneidet und zumindest annähernd zentral auf den Strahlenempfänger (4) trifft, **dadurch gekennzeichnet, dass** der Strahlensender (1) und der Strahlenempfänger (4) Positionen entlang eines jeweiligen Kreisbogens um die gemeinsame Bezugsachse (14) einnehmen können und dass die das Zentren der Kreisbögen auf der gemeinsamen Bezugsachse (14) liegen.

2. Röntgendiagnostikeinrichtung nach Anspruch 1, wobei der Radius des zweiten Kreisbogens unabhängig vom Radius des ersten Kreisbogens variiert werden kann.

3. Röntgendiagnostikeinrichtung nach Anspruche 1 oder 2, wobei die erste und zweite Positioniervorrichtung (2,3) mechanisch gekoppelt sind.

4. Röntgendiagnostikeinrichtung nach einem der Ansprüche 1 bis 3, wobei die erste und zweite Positioniervorrichtung (2,3) elektromechanisch gekoppelt sind.

5. Röntgendiagnostikeinrichtung nach einem der Ansprüche 1 bis 3, wobei die Positionen, die der Strahlensender (1) und der Strahlenempfänger (4) entlang der jeweiligen Kreisbögen einnehmen können, von einer Steuereinrichtung (18) gesteuert eingenommen werden.

6. Röntgendiagnostikeinrichtung nach einem der Ansprüche 1 bis 5, wobei der Bezugsstrahl (13) nach dem Passieren eines zwischen den Positioniervorrichtungen (2,3) angeordneten Untersuchungsobjektes auf den Strahlenempfänger (4) trifft und wobei der Strahlenempfänger (4) Signale erzeugt, die in zum Bezugsstrahl (13) oder zur Bezugsachse (14) lateralen Richtungen ortsaufgelöst sind.

7. Röntgendiagnostikeinrichtung nach einem der Ansprüche 1 bis 6, wobei die Abmessungen des Strahlenempfängers (4) kleiner sind als der mittlere Abstand zwischen der Bezugsachse (14) und dem Bezugsstrahl (13) am Ort des Strahlenempfängers (4) in den verschiedenen, vom Strahlensender (1) eingenommenen Positionen und wobei der Strahlenempfänger (4) so positioniert wird, daß bei den verschiedenen, vom Strahlensender (1) eingenommenen Positionen, der Bezugsstrahl (13) jeweils zumindest annähernd auf das Zentrum des Strahlenempfängers (4) trifft.

## Claims

1. X-ray diagnostic apparatus having a first positioning device (2) with a radiation emitter (1) and having a second positioning device (3) with a radiation receiver (4), which are at a distance from each other, the positioning devices (2, 3) having a common reference axis (14), where when the radiation emitter (1) is coupled to the first positioning device (2), the reference ray (13) of a beam emitted by the radiation emitter (1) intersects the common reference axis (14) at an acute angle α and is incident at least approximately centrally on the radiation receiver (4), **characterized in that** the radiation emitter (1) and the radiation receiver (4) can assume positions along a respective circular arc around the common reference axis (14), and **in that** the centres of the circular arcs lie on the common reference axis (14).

2. X-ray diagnostic apparatus according to Claim 1, it being possible for the radius of the second circular arc to be varied independently of the radius of the first circular arc.

3. X-ray diagnostic apparatus according to Claim 1 or 2, the first and second positioning devices (2, 3) being coupled mechanically.

4. X-ray diagnostic apparatus according to one of Claims 1 to 3, the first and second positioning device (2, 3) being coupled electromechanically.

5. X-ray diagnostic apparatus according to one of Claims 1 to 3, the positions which the radiation emitter (1) and the radiation receiver (4) can assume along the respective circular arcs being assumed under the control of a control device (18).

6. X-ray diagnostic apparatus according to one of Claims 1 to 5, the reference ray (13), after passing through an object to be examined arranged between the positioning devices (2, 3), being incident on the radiation receiver (4), and the radiation receiver (4) generating signals which are locally resolved in directions lateral with respect to the reference ray (13) or to the reference axis (14).

7. X-ray diagnostic apparatus according to one of Claims 1 to 6, the dimensions of the radiation receiver (4) being smaller than the average distance between the reference axis (14) and the reference ray (13) at the location of the radiation receiver (4) in the various positions assumed by the radiation emitter (1), and the radiation receiver (4) being positioned in such a way that, in the various positions assumed by the radiation emitter (1), the reference ray (13) is in each case incident at least approximately on the centre of the radiation receiver (4).

## Revendications

1. Installation de radiodiagnostic comportant un premier dispositif de positionnement (2) ayant un émetteur de rayons (1) et un second dispositif de positionnement (3) ayant un récepteur de rayons (4) qui sont à distance l'un de l'autre, les dispositifs de positionnement (2, 3) possédant un axe de référence (14) commun, le rayon de référence (13) d'un faisceau de rayons émis par l'émetteur de rayons (1), en cas de couplage de l'émetteur de rayons (1) au premier dispositif de positionnement (2), coupant selon un angle aigu α l'axe de référence (14) commun et frappant au moins approximativement au centre le récepteur de rayons (4), **caractérisée en ce que** l'émetteur de rayons (1) et le récepteur de rayons (4) peuvent prendre des positions chacun sur un arc de cercle autour de l'axe de référence (14) commun et que les centres des arcs de cercle se trouvent sur l'axe de référence (14) commun.

2. Installation de radiodiagnostic selon la revendication 1, le rayon du second arc de cercle pouvant varier indépendamment du rayon du premier arc de cercle.

3. Installation de radiodiagnostic selon la revendication 1 ou 2, le premier et le second dispositifs de positionnement (2, 3) étant couplés mécaniquement.

4. Installation de radiodiagnostic selon une des revendications 1 à 3, le premier et le second dispositifs de positionnement (2, 3) étant couplés électromécaniquement.

5. Installation de radiodiagnostic selon une des revendications 1 à 3, les positions, que peuvent adopter l'émetteur de rayons (1) et le récepteur de rayons (4) sur l'arc de cercle considéré, étant prises en étant commandées par un dispositif de commande (18).

6. Installation de radiodiagnostic selon une des revendications 1 à 5, le rayon de référence (13) frappant le récepteur de rayons (4) après être passé par un objet d'examen disposé entre les dispositifs de positionnement (2, 3) et le récepteur de rayons (4) produisant des signaux qui présentent une résolution spatiale dans des directions latérales par rapport au rayon de référence (13) ou à l'axe de référence (14).

7. Installation de radiodiagnostic selon une des revendications 1 à 6, les dimensions du récepteur de rayons (4) étant inférieures à la distance moyenne entre l'axe de référence (14) et le rayon de référence (13) à l'emplacement du récepteur de rayons (4) dans les différentes positions adoptées par l'émetteur de rayons (1) et le récepteur de rayons (4) étant positionné de telle sorte que dans les différentes positions prises par l'émetteur de rayons (1), le rayon de référence (13) frappe au moins approximativement le centre du récepteur de rayons (4).
